(19) Europäisches Patentamt
European Patent Office
Office européen des brevets

(11) EP 1 530 095 B1

(12) EUROPEAN PATENT SPECIFICATION

(45) Date of publication and mention of the grant of the patent:
**03.01.2007 Bulletin 2007/01**

(51) Int Cl.:
***G03G 5/06*** *(2006.01)* ***C07D 417/12*** *(2006.01)*

(21) Application number: **04255902.1**

(22) Date of filing: **28.09.2004**

(54) **Organophotoreceptor with charge transport material having a hydrazone group linked to an epoxy group and a heterocyclic ring**

Organischer Photorezeptor mit Ladungstransportmaterial, das eine Hydrazongruppe mit einer Epoxigruppe verknüpft und einen heterozyklischen Ring enthält

Organophotorécepteur à base de matériau de transport de charge contenant un groupe d'hydrazon et groupe epoxy et une hétérocyclique structure

(84) Designated Contracting States:
**DE FR GB NL**

(30) Priority: **28.10.2003 US 695581**

(43) Date of publication of application:
**11.05.2005 Bulletin 2005/19**

(73) Proprietor: **SAMSUNG ELECTRONICS CO., LTD.**
**Suwon-city,**
**Gyunggi-do (KR)**

(72) Inventors:
- **Jubran, Nusrallah**
  **St. Paul, MN 55119 (US)**
- **Tokarski, Zbigniew**
  **Woodbury, MN 55125 (US)**
- **Grazulevicius, Juozas V.**
  **3032 Kaunas (LT)**
- **Getautis, Vytautas**
  **3031 Kaunas (LT)**
- **Ostrauskaite, Jolita**
  **3000 Raudondavaris Kaunas (LT)**
- **Simokaitiene, Jurate**
  **3000 Kaunas (LT)**
- **Montrimas, Edmundas**
  **2041 Vilnius (LT)**

(74) Representative: **Moy, David et al**
**Appleyard Lees,**
**15 Clare Road**
**Halifax HX1 2HY (GB)**

(56) References cited:
**EP-A- 1 420 303** **DE-A- 3 208 866**
**GB-A- 2 138 159** **US-A- 4 456 671**

- **V. GETAUTIS ET AL.: "Synthesis of branched hydrazones by reaction of N-(2,3-epoxypropyl) derivatives of hydrazones with benzenethiols" CHEMISTRY OF HETEROCYCLIC COMPOUNDS (A TRANSLATION OF KHIMIYA GETEROTSIKLICHESKIKH SOEDINENII), vol. 38, no. 7, July 2002 (2002-07), pages 778-782, XP008045093 PLENUM PRESS CO., NEW YORK, NY.US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to organophotoreceptors suitable for use in electrophotography and, more specifically, to organophotoreceptors having a charge transport material with a hydrazone group linked to an epoxy group and a heterocyclic ring.

In electrophotography, an organophotoreceptor in the form of a plate, disk, sheet, belt, drum or the like having an electrically insulating photoconductive element on an electrically conductive substrate is imaged by first uniformly electrostatically charging the surface of the photoconductive layer, and then exposing the charged surface to a pattern of light. The light exposure selectively dissipates the charge in the illuminated areas where light strikes the surface, thereby forming a pattern of charged and uncharged areas, referred to as a latent image. A liquid or solid toner is then provided in the vicinity of the latent image, and toner droplets or particles deposit in the vicinity of either the charged or uncharged areas to create a toned image on the surface of the photoconductive layer. The resulting toned image can be transferred to a suitable ultimate or intermediate receiving surface, such as paper, or the photoconductive layer can operate as an ultimate receptor for the image. The imaging process can be repeated many times to complete a single image, for example, by overlaying images of distinct color components or effect shadow images, such as overlaying images of distinct colors to form a full color final image, and/or to reproduce additional images.

Both single layer and multilayer photoconductive elements have been used. In single layer embodiments, a charge transport material and charge generating material are combined with a polymeric binder and then deposited on the electrically conductive substrate. In multilayer embodiments, the charge transport material and charge generating material are present in the element in separate layers, each of which can optionally be combined with a polymeric binder, deposited on the electrically conductive substrate. Two arrangements are possible for a two-layer photoconductive element. In one two-layer arrangement (the "dual layer" arrangement), the charge-generating layer is deposited on the electrically conductive substrate and the charge transport layer is deposited on top of the charge generating layer. In an alternate two-layer arrangement (the "inverted dual layer" arrangement), the order of the charge transport layer and charge generating layer is reversed.

In both the single and multilayer photoconductive elements, the purpose of the charge generating material is to generate charge carriers (i.e., holes and/or electrons) upon exposure to light. The purpose of the charge transport material is to accept at least one type of these charge carriers and transport them through the charge transport layer in order to facilitate discharge of a surface charge on the photoconductive element. The charge transport material can be a charge transport compound, an electron transport compound, or a combination of both. When a charge transport compound is used, the charge transport compound accepts the hole carriers and transports them through the layer with the charge transport compound. When an electron transport compound is used, the electron transport compound accepts the electron carriers and transports them through the layer with the electron transport compound.

Although many charge transport materials are known, there is the need for other charge transport materials to meet the various requirements of particular electrophotographic applications.

To produce high quality images, particularly after multiple cycles, it is desirable for the charge transport materials to form a homogeneous solution with the polymeric binder and remain approximately homogeneously distributed through the organophotoreceptor material during the cycling of the material. In addition, it is desirable to increase the amount of charge that the charge transport material can accept (indicated by a parameter known as the acceptance voltage or "$V_{acc}$"), and to reduce retention of that charge upon discharge (indicated by a parameter known as the discharge voltage or "$V_{dis}$").

An aim of the present invention is to provide organophotoreceptors, electrophotographic imaging apparatus, electrophotographic imaging processes, and charge transport materials generally featuring (a) good, and/or beneficial, and/or useful propert(y)ies, or combinations of such properties, and preferably addressing at least one or some of the problems in the art.

There are many charge transport materials available for electrophotography, including, for example, pyrazoline derivatives, fluorene derivatives, oxadiazole derivatives, stilbene derivatives, hydrazone derivatives, carbazole hydrazone derivatives, triaryl amines, polyvinyl carbazole, polyvinyl pyrene, and polyacenaphthylene. However, at least some of the available charge transport materials may suffer some disadvantages and thus there is always the need for charge transport materials to meet the various requirements of electrophotography applications. This represents a further preferred aim of the present invention.

A preferred aim of the present invention is to provide organophotoreceptors having good electrostatic properties.

Moreover, a further preferred aim of the present invention is to provide charge transport materials for organophotoreceptors featuring good mechanical and/or chemical properties, and/or a combination of good mechanical and electrostatic properties.

Suitably, a further aim of embodiments of the present invention is to provide organophotoreceptors and electrophotographic imaging apparatus which can be used to provide high quality of images, even after repeated cycling, electrophotographic apparatus employing the organophotoreceptors and electrophotographic imaging methods using

the organophotoreceptors/charge transport materials.

Other aims and/or advantages of the invention will be set forth in part in the description which follows and, in part, will be apparent or obvious from the description, or may be apparent from, or learned by, practice of the invention.

According to the present invention there is provided an organophotoreceptor, an electrophotographic imaging apparatus, an electrophotographic imaging process, and a charge transport material, as set forth in the appended claims. Preferred features of the invention will be apparent from the dependent claims, and the description which follows.

Accordingly, this invention seeks to provide organophotoreceptors having good electrostatic properties such as high $V_{acc}$ and low $V_{dis}$.

In a first aspect of the present invention there is provided an organophotoreceptor comprising an electrically conductive substrate and a photoconductive element on the electrically conductive substrate, the photoconductive element comprising:

(a) a charge transport material having the formula

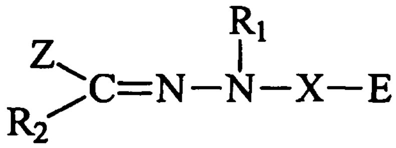

(1)

where $R_1$ and $R_2$ are, independently, H, an alkyl group, an alkaryl group, or an aryl group;

X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;

E is an epoxy group; and

Z comprises a heterocyclic group selected from the group consisting of phenothiazine group, phenoxazine group, phenoxathiin group, dibenzo(1,4)dioxin group, thianthrene group, and phenazine group; and

(b) a charge generating compound.

Preferably, X is a $CH_2$ group.

Preferably, Z is a phenothiazine group.

Preferably, the photoconductive element of the organophotoreceptor further comprises a second charge transport material.

Preferably, the second charge transport material comprises an electron transport compound.

Preferably, the photoconductive element of the organophotoreceptor further comprises a binder.

The organophotoreceptor may be provided, for example, in the form of a plate, a flexible belt, a flexible disk, a sheet, a rigid drum, or a sheet around a rigid or compliant drum. In one embodiment, the organophotoreceptor includes: (a) a photoconductive element comprising the charge transport material, the charge generating compound, a second charge transport material, and a polymeric binder; and (b) the electrically conductive substrate.

In a second aspect of the present invention there is provided an electrophotographic imaging apparatus that comprises (a) a light imaging component; and (b) the organophotoreceptor described herein oriented to receive light from the light imaging component. The apparatus can further comprise a liquid toner dispenser. The method of electrophotographic imaging with photoreceptors containing the charge transport materials noted herein is also described.

In a third aspect of the present invention there is provided an electrophotographic imaging process that includes (a) applying an electrical charge to a surface of the organophotoreceptor described herein; (b) imagewise exposing the surface of the organophotoreceptor to radiation to dissipate charge in selected areas and thereby form a pattern of at least relatively charged and uncharged areas on the surface; (c) contacting the surface with a toner, such as a liquid toner that includes a dispersion of colorant particles in an organic liquid, to create a toned image; and (d) transferring the toned image to a substrate.

In a fourth aspect of the present invention there is provided a charge transport material having the general Formula (1) above, i.e. the formula

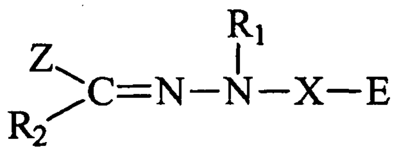

(1)

where $R_1$ and $R_2$ are, independently, H, an alkyl group, an alkaryl group, or an aryl group;

X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;

E is an epoxy group; and

Z comprises a heterocyclic group selected from the group consisting of phenothiazine group, phenoxazine group, phenoxathiin group, dibenzo(1,4)dioxin group, thianthrene group, and phenazine group.

The invention provides suitable charge transport materials for organophotoreceptors featuring a combination of good mechanical and electrostatic properties. The invention generally provides organophotoreceptors having good electrostatic properties, such as high $V_{acc}$ and low $V_{dis}$. These photoreceptors can be used successfully with liquid toners to produce high quality images. The high quality of the imaging system can be maintained after repeated cycling.

Other features and advantages of the invention will be apparent from the following description of the particular embodiments thereof, and from the claims.

Features and embodiments described herein of the first, second, third and fourth aspects of the present invention, respectively, may be regarded as preferred features and embodiments of other aspects of the present invention.

An organophotoreceptor as described herein has an electrically conductive substrate and a photoconductive element comprising a charge generating compound and a charge transport material having a hydrazone group linked through the double bonded carbon to one of a specific set of aromatic heterocyclic groups and linked through the single bonded nitrogen to an epoxy group. These charge transport materials generally have desirable properties as evidenced by their performance in organophotoreceptors for electrophotography. In particular, the charge transport materials of this invention have high charge carrier mobilities and good compatibility with various binder materials, and possess excellent electrophotographic properties. The organophotoreceptors according to this invention generally have a high photosensitivity, a low residual potential, and a high stability with respect to cycle testing, crystallization, and organophotoreceptor bending and stretching. The organophotoreceptors are particularly useful in laser printers and the like as well as fax machines, photocopiers, scanners and other electronic devices based on electrophotography. The use of these charge transport materials is described in more detail below in the context of laser printer use, although their application in other devices operating by electrophotography can be generalized from the discussion below.

As noted above, to produce high quality images, particularly after multiple cycles, it is desirable for the charge transport materials to form a homogeneous solution with the polymeric binder and remain approximately homogeneously distributed through the organophotoreceptor material during the cycling of the material. In addition, it is desirable to increase the amount of charge that the charge transport material can accept (indicated by a parameter known as the acceptance voltage or "$V_{acc}$"), and to reduce retention of that charge upon discharge (indicated by a parameter known as the discharge voltage or "$V_{dis}$").

The charge transport materials can be classified as charge transport compound or electron transport compound. There are many charge transport compounds and electron transport compounds known in the art for electrophotography. Non-limiting examples of charge transport compounds include, for example, pyrazoline derivatives, fluorene derivatives, oxadiazole derivatives, stilbene derivatives, enamine derivatives, hydrazone derivatives, carbazole hydrazone derivatives, triaryl amines, polyvinyl carbazole, polyvinyl pyrene, polyacenaphthylene, or multi-hydrazone compounds comprising at least two hydrazone groups and at least two groups selected from the group consisting of p-(N,N-disubstituted) arylamine such as triphenylamine and heterocycles such as carbazole, julolidine, phenothiazine, phenazine, phenoxazine, phenoxathiin, thiazole, oxazole, isoxazole, dibenzo(1,4)dioxin, thianthrene, imidazole, benzothiazole, benzotriazole, benzoxazole, benzimidazole, quinoline, isoquinoline, quinoxaline, indole, indazole, pyrrole, purine, pyridine, pyridazine, pyrimidine, pyrazine, triazole, oxadiazole, tetrazole, thiadiazole, benzisoxazole, benzisothiazole, dibenzofuran, dibenzothiophene, thiophene, thianaphthene, quinazoline, or cinnoline.

Non-limiting examples of electron transport compounds include, for example, bromoaniline, tetracyanoethylene, tetracyanoquinodimethane, 2,4,7-trinitro-9-fluorenone, 2,4,5,7-tetranitro-9-fluorenone, 2,4,5,7-tetranitroxanthone, 2,4,8-trinitrothioxanthone, 2,6,8-trinitro-indeno4H-indeno[1,2-b]thiophene-4-one, and 1,3,7-trinitrodibenzo thiophene-5,5-dioxide, (2,3-diphenyl-1-indenylidene)malononitrile, 4H-thiopyran-1,1-dioxide and its derivatives such as 4-dicyanomethylene-2,6-diphenyl-4H-thiopyran-1,1-dioxide, 4-dicyanomethylene-2,6-di-m-tolyl-4H-thiopyran-1,1-dioxide, and unsymmetrically substituted 2,6-diaryl-4H-thiopyran-1,1-dioxide such as 4H-1,1-dioxo-2-(p-isopropylphenyl)-6-phenyl-4-(dicyanomethylidene)thiopyran and 4H-1,1-dioxo-2-(p-isopropylphenyl)-6-(2-thienyl)-4-(dicyanomethylidene)thiopyran, derivatives of phospha-2,5-cyclohexadiene, alkoxycarbonyl-9-fluorenylidene)malononitrile derivatives such as (4-n-butoxycarbonyl-9-fluorenylidene)malononitrile, (4-phenethoxycarbonyl-9-fluorenylidene)malononitrile, (4-carbitoxy-9-fluorenylidene)malononitrile, and diethyl(4-n-butoxycarbonyl-2,7-dinitro-9-fluorenylidene)-malonate, anthraquinodimethane derivatives such as 11,11,12,12-tetracyano-2-alkylanthraquinodimethane and 11,11-dicyano-12,12-bis (ethoxycarbonyl)anthraquinodimethane, anthrone derivatives such as 1-chloro-10-[bis(ethoxycarbonyl)methylene]anthrone, 1,8-dichloro-10-[bis(ethoxy carbonyl) methylene]anthrone, 1,8-dihydroxy-10-[bis(ethoxycarbonyl)methylene] anthrone, and 1-cyano-10-[bis(ethoxycarbonyl)methylene]anthrone, 7-nitro-2-aza-9-fluorenylidenemalononitrile, diphenoquinone derivatives, benzoquinone derivatives, naphtoquinone derivatives, quinine derivatives, tetracyanoethylenecyanoethylene, 2,4,8-trinitrothioxantone, dinitrobenzene derivatives, dinitroanthracene derivatives, dinitroacridine derivatives, nitroanthraquinone derivatives, dinitroanthraquinone derivatives, succinic anhydride, maleic anhydride, dibromomaleic anhydride, pyrene derivatives, carbazole derivatives, hydrazone derivatives, N,N-dialkylaniline derivatives, diphenylamine derivatives, triphenylamine derivatives, triphenylmethane derivatives, tetracyanoquinoedimethane, 2,4,5,7-tetranitro-9-fluorenone, 2,4,7-trinitro-9-dicyanomethylene fluorenone, 2,4,5,7-tetranitroxanthone derivatives, and 2,4,8-trinitrothioxanthone derivatives. In some embodiments of interest, the electron transport compound comprises an (alkoxyearbonyl-9-fluorenylidene)malononitrile derivative, such as (4-n-butoxycarbonyl-9-fluorenylidene)malononitrile.

Although there are many charge transport materials available, there remains, as noted above, the is a need for other charge transport materials to meet the various requirements of particular electrophotography applications.

In electrophotography applications, a charge-generating compound within an organophotoreceptor absorbs light to form electron-hole pairs. These electrons and holes can be transported over an appropriate time frame under a large electric field to discharge locally a surface charge that is generating the field. The discharge of the field at a particular location results in a surface charge pattern that essentially matches the pattern drawn with the light. This charge pattern then can be used to guide toner deposition. The charge transport materials described herein are generally especially effective at transporting charge, and in particular electrons from the electron-hole pairs formed by the charge generating compound. In some embodiments, a specific electron transport compound or charge transport compound can also be used along with the charge transport material of this invention.

The layer or layers of materials containing the charge generating compound and the charge transport materials are within an organophotoreceptor. To print a two dimensional image using the organophotoreceptor, the organophotoreceptor has a two dimensional surface for forming at least a portion of the image. The imaging process then continues by cycling the organophotoreceptor to complete the formation of the entire image and/or for the processing of subsequent images.

The organophotoreceptor may be provided in the form of a plate, a flexible belt, a disk, a rigid drum, a sheet around a rigid or compliant drum, or the like. The charge transport material can be in the same layer as the charge generating compound and/or in a different layer from the charge generating compound. Additional layers can be used also, as described further below.

In some embodiments, the organophotoreceptor material comprises, for example: (a) a charge transport layer comprising the charge transport material and a polymeric binder; (b) a charge generating layer comprising the charge generating compound and a polymeric binder; and (c) the electrically conductive substrate. The charge transport layer may be intermediate between the charge generating layer and the electrically conductive substrate. Alternatively, the charge generating layer may be intermediate between the charge transport layer and the electrically conductive substrate. In further embodiments, the organophotoreceptor material has a single layer with both a charge transport material and a charge generating compound within a polymeric binder.

The organophotoreceptors can be incorporated into an electrophotographic imaging apparatus, such as laser printers. In these devices, an image is formed from physical embodiments and converted to a light image that is scanned onto the organophotoreceptor to form a surface latent image. The surface latent image can be used to attract toner onto the surface of the organophotoreceptor, in which the toner image is the same or the negative of the light image projected onto the organophotoreceptor. The toner can be a liquid toner or a dry toner. The toner is subsequently transferred, from the surface of the organophotoreceptor, to a receiving surface, such as a sheet of paper. After the transfer of the toner, the entire surface is discharged, and the material is ready to cycle again. The imaging apparatus can further comprise, for example, a plurality of support rollers for transporting a paper receiving medium and/or for movement of the photoreceptor, a light imaging component with suitable optics to form the light image, a light source, such as a laser, a toner source and delivery system and an appropriate control system.

An electrophotographic imaging process generally can comprise (a) applying an electrical charge to a surface of the organophotoreceptor described herein; (b) imagewise exposing the surface of the organophotoreceptor to radiation to dissipate charge in selected areas and thereby form a pattern of charged and uncharged areas on the surface; (c)

exposing the surface with a toner, such as a liquid toner that includes a dispersion of colorant particles in an organic liquid to create a toner image, to attract toner to the charged or discharged regions of the organophotoreceptor; and (d) transferring the toner image to a substrate.

As described herein, in an aspect of the present invention, for example in the first aspect herein, an organophotoreceptor comprises a charge transport material having the formula

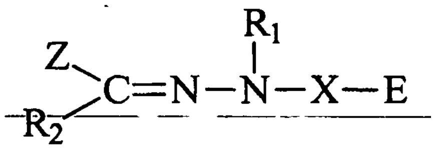

where $R_1$ and $R_2$ are, independently, H, an alkyl group, an alkaryl group, or an aryl group;
X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;
E is an epoxy group; and
Z comprises a heterocyclic group selected from the group consisting of phenothiazine group, phenoxazine group, phenoxathiin group, dibenzo(1,4)dioxin group, thianthrene group, and phenazine group.

In some preferred embodiments, $R_1$ is an aryl group.

In some preferred embodiments, $R_2$ is hydrogen. In yet further preferred embodiments, $R_1$ is an aryl group and $R_2$ is hydrogen.

In some preferred embodiments, $R_1$ is a phenyl group.

In some embodiments, m is an integer between 1 and 10 inclusive, in further embodiments between 1 and 5 inclusive, notably m is 1, and preferably no methylene groups in the group $-(CH_2)_m-$ are replaced.

Preferably, X is a $CH_2$ group.

Preferably, Z is a phenothiazine group.

In some preferred embodiments, X is a $CH_2$ group and Z is a phenothiazine group.

Preferably, the photoconductive element of the organophotoreceptor further comprises a second charge transport material.

Preferably, the second charge transport material comprises an electron transport compound.

Preferably, the photoconductive element of the organophotoreceptor further comprises a binder.

Substitution is liberally allowed on the chemical groups to affect various physical effects on the properties of the compounds, such as mobility, sensitivity, solubility, stability, and the like, as is known generally in the art. In the description of chemical substituents, there are certain practices common to the art that are reflected in the use of language. The term group indicates that the generically recited chemical entity (e.g., alkyl group, phenyl group, aromatic group, vinyl group, etc.) may have any substituent thereon which is consistent with the bond structure of that group. For example, where the term 'alkyl group' is used, that term would not only include unsubstituted linear, branched and cyclic alkyls, such as methyl, ethyl, isopropyl, tert-butyl, cyclohexyl, dodecyl and the like, but also substituents such as hydroxyethyl, cyanobutyl, 1,2,3-trichloropropane, and the like. However, as is consistent with such nomenclature, no substitution would be included within the term that would alter the fundamental bond structure of the underlying group. For example, where a phenyl group is recited, substitution such as 1-hydroxyphenyl, 2,4-fluorophenyl, orthocyanophenyl, 1,3,5-trimethoxyphenyl and the like would be acceptable within the terminology, while substitution of 1,1,2,2,3,3-hexamethylphenyl would not be acceptable as that substitution would require the ring bond structure of the phenyl group to be altered to a non-aromatic form because of the substitution. An aromatic group is a group comprising a 4n+2 pi electron system where n is any integer. When referring to an aromatic group, the substituent cited will include any substitution that does not substantively alter the chemical nature of the 4n+2 pi electron system in the aromatic group. Where the term moiety is used, such as alkyl moiety or phenyl moiety, that terminology indicates that the chemical material is not substituted. Where the term alkyl moiety is used, that term represents only an unsubstituted alkyl hydrocarbon group, whether branched, straight chain, or cyclic.

In general, and except where stated otherwise herein, the following apply. Preferred alkyl groups are $C_{1-16}$ alkyl, especially $C_{1-12}$ alkyl, more especially $C_{1-8}$ alkyl, even more especially $C_{1-4}$ alkyl, suitably substituted or unsubstituted, suitably linear, branched or cyclic.

Organophotoreceptors

The organophotoreceptor may be, for example, in the form of a plate, a sheet, a flexible belt, a disk, a rigid drum, or a sheet around a rigid or compliant drum, with flexible belts and rigid drums generally being used in commercial embodiments. The organophotoreceptor may comprise, for example, an electrically conductive substrate and on the electrically conductive substrate a photoconductive element in the form of one or more layers. The photoconductive element can comprise both a charge transport material and a charge generating compound in a polymeric binder, which may or may not be in the same layer, as well as a second charge transport material such as a charge transport compound or an electron transport compound in some embodiments. For example, the charge transport material and the charge generating compound can be in a single layer. In other embodiments, however, the photoconductive element comprises a bilayer construction featuring a charge generating layer and a separate charge transport layer. The charge generating layer may be located intermediate between the electrically conductive substrate and the charge transport layer. Alternatively, the photoconductive element may have a structure in which the charge transport layer is intermediate between the electrically conductive substrate and the charge generating layer.

The electrically conductive substrate may be flexible, for example in the form of a flexible web or a belt, or inflexible, for example in the form of a drum. A drum can have a hollow cylindrical structure that provides for attachment of the drum to a drive that rotates the drum during the imaging process. Typically, a flexible electrically conductive substrate comprises an electrically insulating substrate and a thin layer of electrically conductive material onto which the photoconductive material is applied.

The electrically insulating substrate may be paper or a film forming polymer such as polyester (e.g., polyethylene terephthalate or polyethylene naphthalate), polyimide, polysulfone, polypropylene, nylon, polyester, polycarbonate, polyvinyl resin, polyvinyl fluoride, polystyrene and the like. Specific examples of polymers for supporting substrates included, for example, polyethersulfone (Stabar™ S-100, available from ICI), polyvinyl fluoride (Tedlar®, available from E.I. DuPont de Nemours & Company), polybisphenol-A polycarbonate (Malaofol™, available from Mobay Chemical Company) and amorphous polyethylene terephthalate (Melinar™, available from ICI Americas, Inc.). The electrically conductive materials may be graphite, dispersed carbon black, iodine, conductive polymers such as polypyroles and Calgon® conductive polymer 261 (commercially available from Calgon Corporation, Inc., Pittsburgh, Pa.), metals such as aluminum, titanium, chromium, brass, gold, copper, palladium, nickel, or stainless steel, or metal oxide such as tin oxide or indium oxide. In embodiments of particular interest, the electrically conductive material is aluminum. Generally, the photoconductor substrate has a thickness adequate to provide the required mechanical stability. For example, flexible web substrates generally have a thickness from about 0.01 mm to about 1 mm, while drum substrates generally have a thickness from about 0.5 mm to about 2 mm.

The charge generating compound is a material that is capable of absorbing light to generate charge carriers, such as a dye or pigment. Non-limiting examples of suitable charge generating compounds include, for example, metal-free phthalocyanines (e.g., ELA 8034 metal-free phthalocyanine available from H.W. Sands, Inc. or Sanyo Color Works, Ltd., CGM-X01), metal phthalocyanines such as titanium phthalocyanine, copper phthalocyanine, oxytitanium phthalocyanine (also referred to as titanyl oxyphthalocyanine, and including any crystalline phase or mixtures of crystalline phases that can act as a charge generating compound), hydroxygallium phthalocyanine, squarylium dyes and pigments, hydroxy-substituted squarylium pigments, perylimides, polynuclear quinones available from Allied Chemical Corporation under the tradename Indofast® Double Scarlet, Indofast® Violet Lake B, Indofast® Brilliant Scarlet and Indofast® Orange, quinacridones available from DuPont under the tradename Monastral™ Red, Monastral™ Violet and Monastral™ Red Y, naphthalene 1,4,5,8-tetracarboxylic acid derived pigments including the perinones, tetrabenzoporphyrins and tetranaphthaloporphyrins, indigo- and thioindigo dyes, benzothioxanthene-derivatives, perylene 3,4,9,10-tetracarboxylic acid derived pigments, polyazo-pigments including bisazo-, trisazo- and tetrakisazo-pigments, polymethine dyes, dyes containing quinazoline groups, tertiary amines, amorphous selenium, selenium alloys such as selenium-tellurium, selenium-tellurium-arsenic and selenium-arsenic, cadmium sulphoselenide, cadmium selenide, cadmium sulphide, and mixtures thereof. For some embodiments, the charge generating compound comprises oxytitanium phthalocyanine (e.g., any phase thereof), hydroxygallium phthalocyanine or a combination thereof

The photoconductive layer of this invention may optionally contain a second charge transport material which may be a charge transport compound, an electron transport compound, or a combination of both. Generally, any charge transport compound or electron transport compound known in the art can be used as the second charge transport material.

An electron transport compound and a UV light stabilizer can have a synergistic relationship for providing desired electron flow within the photoconductor. The presence of the UV light stabilizers alters the electron transport properties of the electron transport compounds to improve the electron transporting properties of the composite. UV light stabilizers can be ultraviolet light absorbers or ultraviolet light inhibitors that trap free radicals.

UV light absorbers can absorb ultraviolet radiation and dissipate it as heat. UV light inhibitors are thought to trap free radicals generated by the ultraviolet light and after trapping of the free radicals, subsequently to regenerate active stabilizer moieties with energy dissipation. In view of the synergistic relationship of the UV stabilizers with electron

transport compounds, the particular advantages of the UV stabilizers may not be their UV stabilizing abilities, although the UV stabilizing ability may be further advantageous in reducing degradation of the organophotoreceptor over time. The improved synergistic performance of organophotoreceptors with layers comprising both an electron transport compound and a UV stabilizer are described further in copending U.S. Patent Application Serial Number 10/425,333 filed on April 28, 2003 to Zhu, entitled "Organophotoreceptor With A Light Stabilizer," published as US 2003/0228534 A1.

Non-limiting examples of suitable light stabilizer include, for example, hindered trialkylamines such as Tinuvin 144 and Tinuvin 292 (from Ciba Specialty Chemicals, Terrytown, NY), hindered alkoxydialkylamines such as Tinuvin 123 (from Ciba Specialty Chemicals), benzotriazoles such as Tinuvan 328, Tinuvin 900 and Tinuvin 928 (from Ciba Specialty Chemicals), benzophenones such as Sanduvor 3041 (from Clariant Corp., Charlotte, N.C.), nickel compounds such as Arbestab (from Robinson Brothers Ltd, West Midlands, Great Britain), salicylates, cyanocinnamates, benzylidene malonates, benzoates, oxanilides such as Sanduvor VSU (from Clariant Corp., Charlotte, N.C.), triazines such as Cyagard UV-1164 (from Cytec Industries Inc., N.J.), polymeric sterically hindered amines such as Luchem (from Atochem North America, Buffalo, NY). In some embodiments, the light stabilizer is selected from the group consisting of hindered trialkylamines having the following formula:

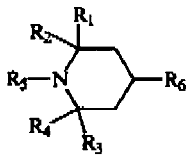 , 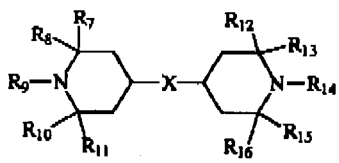 ,

where $R_1$, $R_2$, $R_3$, $R_4$, $R_6$, $R_7$, $R_8$, $R_{10}$, $R_{11}$, $R_{12}$, $R_{13}$, $R_{15}$ and $R_{16}$ are, independently, hydrogen, alkyl group, or ester, or ether group; and $R_5$, $R_9$, and $R_{14}$ are, independently, alkyl group; and X is a linking group selected from the group consisting of $-O-CO-(CH_2)_m-CO-O-$ where m is between 2 to 20.

The binder generally is capable of dispersing or dissolving the charge transport material (in the case of the charge transport layer or a single layer construction), the charge generating compound (in the case of the charge generating layer or a single layer construction) and/or an electron transport compound for appropriate embodiments. Examples of suitable binders for both the charge generating layer and charge transport layer generally include, for example, polystyrene-co-butadiene, polystyrene-co- acrylonitrile, modified acrylic polymers, polyvinyl acetate, styrene-alkyd resins, soya-alkyl resins, polyvinylchloride, polyvinylidene chloride, polyacrylonitrile, polycarbonates, polyacrylic acid, polyacrylates, polymethacrylates, styrene polymers, polyvinyl butyral, alkyd resins, polyamides, polyurethanes, polyesters, polysulfones, polyethers, polyketones, phenoxy resins, epoxy resins, silicone resins, polysiloxanes, poly (hydroxyether) resins, polyhydroxystyrene resins, novolak, poly(phenylglycidyl ether)-co-dicyclopentadiene, copolymers of monomers used in the above-mentioned polymers, and combinations thereof. Suitable binders include, for example, polyvinyl butyral, such as BX-1 and BX-5 from Sekisui Chemical Co. Ltd., Japan.

Suitable optional additives for any one or more of the layers include, for example, antioxidants, coupling agents, dispersing agents, curing agents, surfactants and combinations thereof.

The photoconductive element overall typically has a thickness from about 10 microns to about 45 microns. In the dual layer embodiments having a separate charge generating layer and a separate charge transport layer, charge generation layer generally has a thickness from about 0.5 microns to about 2 microns, and the charge transport layer has a thickness from about 5 microns to about 35 microns. In embodiments in which the charge transport material and the charge generating compound are in the same layer, the layer with the charge generating compound and the charge transport composition generally has a thickness from about 7 microns to about 30 microns. In embodiments with a distinct electron transport layer, the electron transport layer has an average thickness from about 0.5 microns to about 10 microns and in further embodiments from about 1 micron to about 3 microns. In general, an electron transport overcoat layer can increase mechanical abrasion resistance, increases resistance to carrier liquid and atmospheric moisture, and decreases degradation of the photoreceptor by corona gases. A person of ordinary skill in the art will recognize that additional ranges of thickness within the explicit ranges above are contemplated and are within the present disclosure.

Generally, for the organophotoreceptors described herein, the charge generation compound is in an amount from about 0.5 to about 25 weight percent, in further embodiments in an amount from about 1 to about 15 weight percent, and in other embodiments in an amount from about 2 to about 10 weight percent, based on the weight of the photoconductive layer. Suitably, the charge transport material is in an amount from about 10 to about 80 weight percent, based on the weight of the photoconductive layer, in further embodiments in an amount from about 35 to about 60 weight percent, and in other embodiments from about 45 to about 55 weight percent, based on the weight of the photoconductive layer. The optional second charge transport material, when present, can be in an amount of at least about 2 weight percent, in other embodiments from about 2.5 to about 25 weight percent, based on the weight of the photoconductive

layer, and in further embodiments in an amount from about 4 to about 20 weight percent, based on the weight of the photoconductive layer. Suitably, the binder is in an amount from about 15 to about 80 weight percent, based on the weight of the photoconductive layer, and in further embodiments in an amount from about 20 to about 75 weight percent, based on the weight of the photoconductive layer. A person of ordinary skill in the art will recognize that additional ranges within the explicit ranges of compositions are contemplated and are within the present disclosure.

For the dual layer embodiments with a separate charge generating layer and a charge transport layer, the charge generation layer generally comprises a binder in an amount from about 10 to about 90 weight percent, in further embodiments from about 15 to about 80 weight percent and in some embodiments in an amount from about 20 to about 75 weight percent, based on the weight of the charge generation layer. The optional charge transport material in the charge generating layer, if present, generally can be in an amount of at least about 2.5 weight percent, in further embodiments from about 4 to about 30 weight percent and in other embodiments in an amount from about 10 to about 25 weight percent, based on the weight of the charge generating layer. The charge transport layer generally comprises a binder in an amount from about 20 weight percent to about 70 weight percent and in further embodiments in an amount from about 30 weight percent to about 50 weight percent. A person of ordinary skill in the art will recognize that additional ranges of binder concentrations for the dual layer embodiments within the explicit ranges above are contemplated and are within the present disclosure.

For the embodiments with a single layer having a charge generating compound and a charge transport material, the photoconductive layer generally comprises a binder, a charge transport material and a charge generation compound. The charge generation compound can be in an amount from about 0.05 to about 25 weight percent and in further embodiment in an amount from about 2 to about 15 weight percent, based on the weight of the photoconductive layer. The charge transport material can be in an amount from about 10 to about 80 weight percent, in other embodiments from about 25 to about 65 weight percent, in additional embodiments from about 30 to about 60 weight percent and in further embodiments in an amount of from about 35 to about 55 weight percent, based on the weight of the photoconductive layer, with the remainder of the photoconductive layer comprising the binder, and optionally additives, such as any conventional additives. A single layer with a charge transport composition and a charge generating compound generally comprises a binder in an amount from about 10 weight percent to about 75 weight percent, in other embodiments from about 20 weight percent to about 60 weight percent, and in further embodiments from about 25 weight percent to about 50 weight percent. Optionally, the layer with the charge generating compound and the charge transport material may comprise a second charge transport material. The optional second charge transport material, if present, generally can be in an amount of at least about 2.5 weight percent, in further embodiments from about 4 to about 30 weight percent and in other embodiments in an amount from about 10 to about 25 weight percent, based on the weight of the photoconductive layer. A person of ordinary skill in the art will recognize that additional composition ranges within the explicit compositions ranges for the layers above are contemplated and are within the present disclosure.

In general, any layer with an electron transport layer can advantageously further include a UV light stabilizer. In particular, the electron transport layer generally can comprise an electron transport compound, a binder and an optional UV light stabilizer. An overcoat layer comprising an electron transport compound is described further in copending U.S. Patent Application Serial No. 10/396,536 to Zhu et al. entitled, "Organophotoreceptor With An Electron Transport Layer," published as US 2003/0194626 A1. For example, an electron transport compound as described above may be used in the release layer of the photoconductors described herein. Suitably, the electron transport compound in an electron transport layer can be in an amount from about 10 to about 50 weight percent, and in other embodiments in an amount from about 20 to about 40 weight percent, based on the weight of the electron transport layer. A person of ordinary skill in the art will recognize that additional ranges of compositions within the explicit ranges are contemplated and are within the present disclosure.

The UV light stabilizer, if present, in any one or more appropriate layers of the photoconductor generally is suitably in an amount from about 0.5 to about 25 weight percent and in some embodiments in an amount from about 1 to about 10 weight percent, based on the weight of the particular layer. A person of ordinary skill in the art will recognize that additional ranges of compositions within the explicit ranges are contemplated and are within the present disclosure.

For example, the photoconductive layer may be formed by dispersing or dissolving the components, such as one or more of a charge generating compound, the charge transport material of this invention, a second charge transport material such as a charge transport compound or an electron transport compound, a UV light stabilizer, and a polymeric binder in organic solvent, coating the dispersion and/or solution on the respective underlying layer and drying the coating. In particular, the components can be dispersed by high shear homogenization, ball-milling, attritor milling, high energy bead (sand) milling or other size reduction processes or mixing means known in the art for effecting particle size reduction in forming a dispersion.

The photoreceptor may optionally have one or more additional layers as well. An additional layer can be, for example, a sub-layer or an overcoat layer, such as a barrier layer, a release layer, a protective layer, or an adhesive layer. A release layer or a protective layer may form the uppermost layer of the photoconductor element. A barrier layer may be sandwiched between the release layer and the photoconductive element or used to overcoat the photoconductive

element. The barrier layer provides protection from abrasion to the underlayers. An adhesive layer locates and improves the adhesion between a photoconductive element, a barrier layer and a release layer, or any combination thereof. A sub-layer is a charge blocking layer and locates between the electrically conductive substrate and the photoconductive element. The sub-layer may also improve the adhesion between the electrically conductive substrate and the photoconductive element.

Suitable barrier layers include, for example, coatings such as crosslinkable siloxanol-colloidal silica coating and hydroxylated silsesquioxane-colloidal silica coating, and organic binders such as polyvinyl alcohol, methyl vinyl ether/maleic anhydride copolymer, casein, polyvinyl pyrrolidone, polyacrylic acid, gelatin, starch, polyurethanes, polyimides, polyesters, polyamides, polyvinyl acetate, polyvinyl chloride, polyvinylidene chloride, polycarbonates, polyvinyl butyral, polyvinyl acetoacetal, polyvinyl formal, polyacrylonitrile, polymethyl methacrylate, polyacrylates, polyvinyl carbazoles, copolymers of monomers used in the above-mentioned polymers, vinyl chloride/vinyl acetate/vinyl alcohol terpolymers, vinyl chloride/vinyl acetate/maleic acid terpolymers, ethylene/vinyl acetate copolymers, vinyl chloride/vinylidene chloride copolymers, cellulose polymers, and mixtures thereof. The above barrier layer polymers optionally may contain small inorganic particles such as fumed silica, silica, titania, alumina, zirconia, or a combination thereof. Barrier layers are described further in U.S. Patent 6,001,522 to Woo et al., entitled "Barrier Layer For Photoconductor Elements Comprising An Organic Polymer And Silicas,". The release layer topcoat may comprise any release layer composition known in the art. In some embodiments, the release layer is a fluorinated polymer, siloxane polymer, fluorosilicone polymer, silane, polyethylene, polypropylene, polyacrylate, or a combination thereof. The release layers can comprise crosslinked polymers.

The release layer may comprise, for example, any release layer composition known in the art. In some embodiments, the release layer comprises a fluorinated polymer, siloxane polymer, fluorosilicone polymer, polysilane, polyethylene, polypropylene, polyacrylate, poly(methyl methacrylate-co-methacrylic acid), urethane resins, urethane-epoxy resins, acrylated-urethane resins, urethane-acrylic resins, or a combination thereof. In further embodiments, the release layers comprise crosslinked polymers.

The protective layer can protect the organophotoreceptor from chemical and mechanical degradation. The protective layer may comprise any protective layer composition known in the art. In some embodiments, the protective layer is a fluorinated polymer, siloxane polymer, fluorosilicone polymer, polysilane, polyethylene, polypropylene, polyacrylate, poly(methyl methacrylate-co-methacrylic acid), urethane resins, urethane-epoxy resins, acrylated-urethane resins, urethane-acrylic resins, or a combination thereof. In some embodiments of particular interest, the protective layers are crosslinked polymers.

An overcoat layer may comprise an electron transport compound as described further in copending U.S. Patent Application Serial No. 10/396,536, filed on March 25, 2003 to Zhu et al. entitled, "Organoreceptor With An Electron Transport Layer," published as US 2003/0194626 A1. For example, an electron transport compound, as described above, may be used in the overcoat layer of this invention. Suitably, the electron transport compound in the overcoat layer can be in an amount from about 2 to about 50 weight percent, and in other embodiments in an amount from about 10 to about 40 weight percent, based on the weight of the overcoat layer. A person of ordinary skill in the art will recognize that additional ranges of composition within the explicit ranges are contemplated and are within the present disclosure.

Generally, adhesive layers comprise a film forming polymer, such as polyester, polyvinylbutyral, polyvinylpyrolidone, polyurethane, polymethyl methacrylate, poly(hydroxy amino ether) and the like. Barrier and adhesive layers are described further in U.S. Patent 6,180,305 to Ackley et al., entitled "Organic Photoreceptors for Liquid Electrophotography,".

Sub-layers can comprise, for example, polyvinylbutyral, organosilanes, hydrolyzable silanes, epoxy resins, polyesters, polyamides, polyurethanes, and the like. In some embodiments, the sub-layer has a dry thickness between about 20 Angstroms and about 2,000 Angstroms. Sublayers containing metal oxide conductive particles can be between about 1 and about 25 microns thick. A person of ordinary skill in the art will recognize that additional ranges of compositions and thickness within the explicit ranges are contemplated and are within the present disclosure.

The charge transport materials as described herein, and photoreceptors including these compounds, are suitable for use in an imaging process with either dry or liquid toner development. For example, any dry toners and liquid toners known in the art may be used in the process and the apparatus of this invention. Liquid toner development can be desirable because it offers the advantages of providing higher resolution images and requiring lower energy for image fixing compared to dry toners. Examples of suitable liquid toners are known in the art. Liquid toners generally comprise toner particles dispersed in a carrier liquid. The toner particles can comprise a colorant/pigment, a resin binder, and/or a charge director. In some embodiments of liquid toner, a resin to pigment ratio can be from 1:1 to 10:1, and in other embodiments, from 4:1 to 8:1. Liquid toners are described further in Published U.S. Patent Applications 2002/0128349, entitled "Liquid Inks Comprising A Stable Organosol," 2002/0086916, entitled "Liquid Inks Comprising Treated Colorant Particles," and 2002/0197552, entitled "Phase Change Developer For Liquid Electraphatography,".

Charge Transport Material

As described herein, an organophotoreceptor comprises a charge transport material having the formula

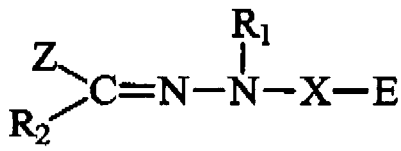

(1)

where $R_1$ and $R_2$ are, independently, H, an alkyl group, an alkaryl group, or an aryl group;

X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;

E is an epoxy group; and

Z comprises a heterocyclic group selected from the group consisting of a phenothiazine group, a phenoxazine group, a phenoxathiin group, a dibenzo(1,4)dioxin group, a thianthrene group, and a phenazine group.

In some preferred embodiments, $R_1$ is an aryl group.

In some preferred embodiments, $R_2$ is hydrogen. In yet further preferred embodiments, $R_1$ is an aryl group and $R_2$ is hydrogen.

In some preferred embodiments, $R_1$ is a phenyl group.

In some embodiments, m is an integer between 1 and 10 inclusive, in further embodiments between 1 and 5 inclusive, notably m is 1, and preferably no methylene groups in the group $-(CH_2)_m-$ are replaced.

Preferably, X is a $CH_2$ group.

Preferably, Z is a phenothiazine group.

In some preferred embodiments, X is a $CH_2$ group and Z is a phenothiazine group.

Specific, non-limiting examples of suitable charge transport materials within the general Formula (1) of the present invention have the following structures (2) - (3):

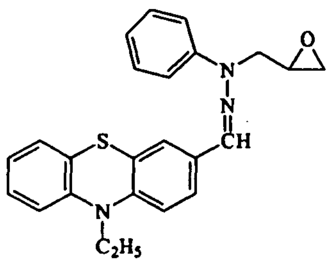

(2),

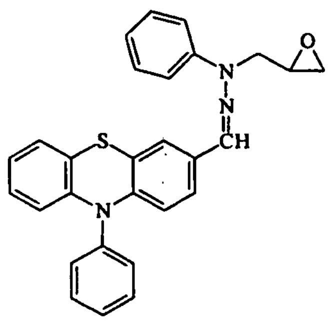

(3).

Synthesis Of Charge Transport Materials

The synthesis of the charge transport materials of this invention can be prepared by the following multi-step synthetic procedure, although other suitable procedures can be used by a person of ordinary skill in the art based on the disclosure herein.

**Step 1: Substitution Of Heterocyclic Group**

A mixture of a heterocycle, such as phenothiazine, phenoxazine, phenoxathiin, dibenzo(1,4)dioxin, thianthrene, and phenazine, an iodo-compound, such as iodoalkane, iodoaryl or iodoalkaryl compound, potassium hydroxide (KOH), and tetra-n-butylammonium hydrogen sulfate in dry toluene is refluxed for 24 hours. The cooled reaction mixture is

filtered and the solvent is evaporated. The product is a corresponding substituted heterocycle which may be crystallized from a solvent such as methanol, with the alkyl, aryl or alkaryl substitutent added at one of the heteroatoms of the heterocycle.

**Step 2: Monoformylation Of The Substituted Heterocycle**

Phosphorus oxychloride ($POCl_3$) is added dropwise to dry dimethylformamide (DMF) at 0 °C under a nitrogen atmosphere. This solution is warmed up slowly to room temperature. A solution of the substituted heterocycle of step 1 in dry DMF is added dropwise to the solution. The reaction mixture is refluxed at 80 °C for 24 hours and then poured into ice water. This solution is neutralized with potassium hydroxide until pH reaches 6-8. The product is extracted with chloroform. The chloroform extract is dried with anhydrous sodium sulfate, filtered and distilled. The product is a monoformyl derivative of the substituted heterocycle which may be crystallized from a solvent such as methanol.

**Step 3: Reaction Of Monoformyl Derivative With A Hydrazine**

The monoformyl derivative obtained in step 2 above is dissolved in methanol under mild heating. Then, the reaction mixture is cooled. A solution of N-phenylhydrazine in methanol is added to the cooled reaction mixture. The reaction mixture is refluxed for 0.5 hour. The precipitated product is a hydrazone derivative which is filtered, washed with a large amount of methanol, and then dried.

**Step 4: Reaction Of Hydrazone Derivative With Epichlorohydrin**

The hydrazone derivative is dissolved in epichlorohydrin. Then, KOH is added to the reaction mixture in three portions. In addition, anhydrous sodium sulfate is added during the first addition of KOH. The reaction mixture is stirred at 30 °C for 24 hours. The crude product is extracted with diethyl ether. The solvent and epichlorohydrin are evaporated in vacuum. The final product, an epoxy substituted compound, is purified by column chromatography with silica gel and an eluant mixture of ethyl acetate and n-hexane in a volume ration of 1:3.

While epichlorohydrin can be used to form the epoxy substituted compound with X = $-CH_2-$, alternatively other X groups can be formed, for example, using a difunctional compound with a halogen and a vinyl group (C=C) with or without substitution. The halide group can be replaced by a bond to the single bonded nitrogen atom of the hydrazone group by a nucleophilic substitution. The vinyl can be converted to the epoxy group in an epoxidation reaction, for example, by the reaction with perbenzoic acid or other peroxy acid, in an electrophilic addition reaction. Thus, the identity of X can be selected as desired through the introduction of the difunctional compound.

The invention will now be described further by way of the following illustrative examples. These examples are to be viewed as being illustrative of specific materials falling within the broader disclosure presented above.

**EXAMPLES**

Example 1 - <u>Synthesis And Characterization Charge Transport Materials</u>

This example described the synthesis and characterization of Compounds 2-3 in which the numbers refer to formula numbers above. The characterization involves both chemical characterization and the electronic characterization of materials formed with the compound.

<u>Compound (2)</u>

**10-Ethylphenothiazine.** A mixture of 10 g (0.05 mol) of phenothiazine, 11.7 g (0.075mol) of iodoethane, 4.2 g (0.075 mol) of potassium hydroxide and 0.25 g of tetra-n-butylammonium hydrogen sulfate in 200 ml of dry toluene was refluxed for 24 hours. After cooling, the reaction mixture was filtered, and the solvent was evaporated. The product was crystallized from methanol. The yield of 10-ethylphenothiazine ($C_{14}H_{13}NS$, FW=227.33) was 90%.

**10-Ethylphenothiazine-3-carbaldehyde.** Phosphorus oxychloride ($POCl_3$, 3.7 ml, 0.04 mol) was added dropwise to 4.4 ml (0.06 mol) of dry dimethylformamide (DMF) at 0 °C under a nitrogen atmosphere. This solution was warmed up slowly to room temperature. Then, a solution of 5 g (0.02 mol) of 10-ethylphenothiazine in dry DMF was added dropwise. The reaction mixture was refluxed at 80 °C for 24 hours and poured into the ice water. This solution was neutralized with potassium hydroxide until the pH reached 6-8. The product was extracted with chloroform. The chloroform extract was dried with anhydrous sodium sulfate, filtered and distilled. The product was crystallized from methanol. The yield of 10-ethylphenothiazine-3-carbaldehyde ($C_{15}H_{13}NOS$, FW=255.34) was 65%.

**10-Ethylphenothiazine-3-carbaldehyde-*N*-phenylhydrazone.**

10-Ethylphenothiazine-3-carbaldehyde (3 g, 0.012 mol) was dissolved in 30 ml of methanol under mild heating. A solution of 1.9 g (0.018 mol) of *N*-phenylhydrazine in methanol was added to the cooled reaction mixture. Then, the reaction mixture was refluxed for 0.5 hour. The precipitated product was filtered, washed with a large amount of methanol and then dried. The yield of yellowish crystals of 10-ethylphenothiazine-3-carbaldehyde-*N*-phenylhydrazone ($C_{21}H_{19}N_3S$, FW=345.00) was 3 g (75%).

**10-Ethylphenothiazine-3-carbaldehyde-*N*-(2,3- epoxypropyl)-*N*-phenylhydrazone.** 10-Ethylphenothiazine-3-carbaldehyde-*N*-phenylhydrazone (2 g, 0.0058 mol) was dissolved in 4 g (0,043 mol) of epichlorohydrin. A 0.9 g (0.017 mol) quantity of KOH was added to the reaction mixture in three portions. Anhydrous sodium sulfate (0.33 g, 0.0023 mol) was also added during the first addition of KOH. The reaction mixture was stirred at 30 °C for 24 hours. The crude product was extracted with diethyl ether. The solvent and epichlorohydrin were evaporated in vacuum. The crude product was purified by column chromatography with silica gel and an eluant mixture of ethyl acetate and n-hexane in a volume ratio of 1:3. The yield of 10-ethylphenothiazine-3-carbaldehyde-*N*-(2,3-epoxypropyl)-*N*-phenylhydrazone (Compound (2) $C_{24}H_{23}N_3OS$, FW=401.53) was 1.4 g (60%). A $^1$H NMR spectrum yielded the following ($CDCl_3$, δ, ppm): 1.3 (t, 3H, ($-CH_3$)); 2.1 (m, 2H, ($-CH_2$-O-)); 2.9 (k, H, ($-CH_2$-O-)); 3.5-4.0(m, 2H, ($-CH_2$-N-)); 4.1 (q, 2H, 2 ($-CH_2$-N)); 7.3 (q, H, (-CH=N-)); 6.6-7.6 (m, Ar).

Compound (3)

Compound (3) can be obtained similarly according to the procedure for Compound (2) except that iodobenzene is used to replace iodoethane in the first step.

**10-Benzylphenothiazine**. A mixture of phenothiazine (15 g, 0.075 mol), 9.6 g (0.150 mol) of copper powder, 35.3 g (0.26 mol) of potassium carbonate, and 1.98 g (0.0075 mol) of 18-crown-6 in 20 ml of 1,2-dichlorobenzene was reflux for 0.5 hour. Then, 23 g (0.11 mol) of iodobenzene was added slowly, and the reaction mixture was refluxed for 24 hours. Then, inorganic components were removed by filtering the hot reaction mixture. The crude product formed crystals from the reaction mixture. The product was purified by recrystallization from methanol. The yield of 10-benzylphenothiazine ($C_{18}H_{13}NS$, FW=275.00) was 12.4 g (60 %).

Compound (3) was obtained similarly according to Steps 2-4 for Compound (2).

Example 2 - Charge Mobility Measurements

This example describes the measurement of charge mobility for samples formed with the two charge transport materials described in Example 1.

Sample 1

A mixture of 0.1 g of Compound (2) and 0.1 g of polycarbonate Z was dissolved in 2 ml of THF. The solution was coated on a polyester film with conductive aluminum layer by the dip roller method. After drying for 15 min. at 80°C temperature, a clear 10 μm thick layer was formed.

Sample 2

Sample 2 was prepared according to the procedure for Sample 1, except that Compound (3) was used in place of Compound (2).

Mobility Measurements

Each sample was corona charged positively up to a surface potential U and illuminated with 2 ns long nitrogen laser light pulse. The hole mobility μ was determined as described in Kalade et al., "Investigation of charge carrier transfer in electrophotographic layers of chalkogenide glasses," Proceeding IPCS 1994: The Physics and Chemistry of Imaging Systems, Rochester, NY, pp. 747-752. The hole mobility measurement was repeated, and between measurements changes were made to the charging regime and charging of the sample to different U values, which corresponded to different electric field strength inside the layer E. This dependence on electric field strength was approximated by the formula

$$\mu = \mu_0 e^{\alpha\sqrt{E}}.$$

Here E is electric field strength, $\mu_0$ is the zero field mobility and $\alpha$ is Pool-Frenkel parameter. The mobility characterizing parameters $\mu_0$ and $\alpha$ values as well as the mobility value at the $6.4 \times 10^5$ V/cm field strength as determined from these measurements are given in Table 1.

Table 1

| Sample | $\mu_0$ ($cm^2/V \cdot s$) | $\mu$($cm^2/V \cdot s$) at $6.4 \times 10^5$ V/cm | $\alpha(cm/V)^{1/2}$ |
|---|---|---|---|
| 1 | $1 \cdot 10^{-10}$ | $2.7 \cdot 10^{-8}$ | 0.0070 |
| 2 | $3.3 \cdot 10^{-10}$ | $3.4 \cdot 10^{-8}$ | 0.0058 |

Example 3 - Ionization Potential Measurements

This example describes the measurement of the ionization potential for the two charge transport materials described in Example 1.

To perform the ionization potential measurements, a thin layer of charge transport material about 0.5 $\mu$m thickness was coated from a solution of 2 mg of charge transport material in 0.2 ml of tetrahydrofuran on a 20 $cm^2$ substrate surface. The substrate was polyester film with an aluminum layer over a methylcellulose sublayer of about 0.4 $\mu$m thickness.

Ionization potential was measured as described in Grigalevicius et al., "3,6-Di(N-diphenylamino)-9-phenylcarbazole and its methyl-substituted derivative as novel hole-transporting amorphous molecular materials," Synthetic Metals **128** (2002), p. 127-131. In particular, each sample was illuminated with monochromatic light from the quartz monochromator with a deuterium lamp source. The power of the incident light beam was $2\text{-}5 \cdot 10^{-8}$ W. A negative voltage of -300 V was supplied to the sample substrate. A counter-electrode with the $4.5 \times 15$ $mm^2$ slit for illumination was placed at 8 mm distance from the sample surface. The counter-electrode was connected to the input of a BK2-16 type electrometer, working in the open input regime, for the photocurrent measurement. A $10^{-15}$ - $10^{-12}$ amp photocurrent was flowing in the circuit under illumination. The photocurrent, I, was strongly dependent on the incident light photon energy h$\nu$. The $I^{0.5}=f(h\nu)$ dependence was plotted. Usually, the dependence of the square root of photocurrent on incident light quanta energy is well described by linear relationship near the threshold (see references "Ionization Potential of Organic Pigment Film by Atmospheric Photoelectron Emission Analysis," Electrophotography, 28, Nr. 4, p. 364 (1989) by E. Miyamoto, Y. Yamaguchi, and M. Yokoyama; and "Photoemission in Solids," Topics in Applied Physics, 26, 1-103 (1978) by M. Cordona and L. Ley). The linear part of this dependence was extrapolated to the h$\nu$ axis, and the Ip value was determined as the photon energy at the interception point. The ionization potential measurement has an error of $\pm 0.03$ eV. The ionization potential values are given in Table 2.

Table 2 - Ionization Potential

| Compound | $I_p$ (eV) |
|---|---|
| 2 | 5.38 |
| 3 | 5.37 |

As understood by those skilled in the art, additional substitution, variation among substituents, and alternative methods of synthesis and use may be practiced within the scope and intent of the present disclosure of the invention. The embodiments above are intended to be illustrative and not limiting. Additional embodiments are within the claims. Although the present invention has been described with reference to particular embodiments, workers skilled in the art will recognize that changes may be made in form and detail without departing from the scope of the invention.

Although a few preferred embodiments have been shown and described, it will be appreciated by those skilled in the art that various changes and modifications might be made without departing from the scope of the invention, as defined in the appended claims.

## Claims

1. An organophotoreceptor comprising an electrically conductive substrate and a photoconductive element on the electrically conductive substrate, the photoconductive element comprising:

(a) a charge transport material having the formula

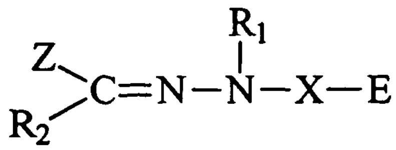

where $R_1$ and $R_2$ are, independently, H, an alkyl group, an alkaryl group, or an aryl group;
X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;
E is an epoxy group; and
Z comprises a phenothiazine group, a phenoxazine group, a phenoxathiin group, a dibenzo(1,4)dioxin group, a thianthrene group, or a phenazine group; and
(b) a charge generating compound.

2. An organophotoreceptor according to claim 1 wherein X is a $CH_2$ group.

3. An organophotoreceptor according to either of claims 1 and 2 wherein Z is a phenothiazine group.

4. An organophotoreceptor according to any preceding claim wherein the charge transport material has a formula selected form the group consisting of the following:

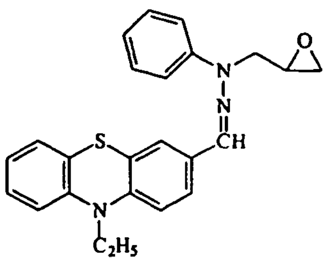
,
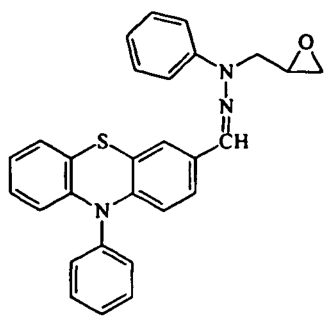
.

5. An organophotoreceptor according to any preceding claim wherein the photoconductive element further comprises a second charge transport material.

6. An organophotoreceptor according to claim 5 wherein the second charge transport material comprises an electron transport compound.

7. An organophotoreceptor according to any preceding claim wherein the photoconductive element further comprises a binder.

8. An electrophotographic imaging apparatus comprising:

(a) a light imaging component; and

(b) an organophotoreceptor oriented to receive light from the light imaging component, the organophotoreceptor comprising an electrically conductive substrate and a photoconductive element on the electrically conductive substrate, the photoconductive element comprising:

(i) a charge transport material having the formula

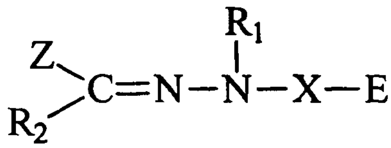

where $R_1$ and $R_2$ are, independently, H, an alkyl group, an alkaryl group, or an aryl group;
X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;
E is an epoxy group; and
Z comprises a phenothiazine group, a phenoxazine group, a phenoxathiin group, a dibenzo(1,4)dioxin group, a thianthrene group, or a phenazine group; and
(ii) a charge generating compound.

**9.** An electrophotographic imaging apparatus according to claim 8 wherein X is a $CH_2$ group.

**10.** An electrophotographic imaging apparatus according to either of claims 8 and 9 wherein Z is a phenothiazine group.

**11.** An electrophotographic imaging apparatus according to any of claims 8 to 10, wherein the charge transport material has a formula selected form the group consisting of the following:

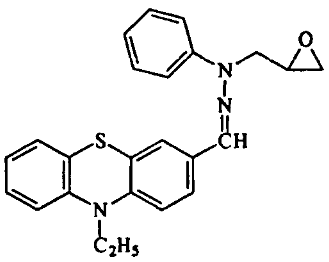

,

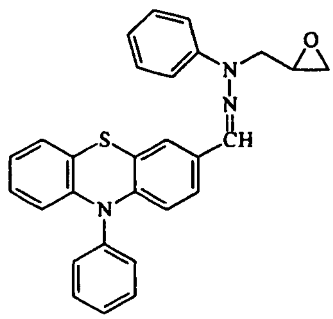

.

**12.** An electrophotographic imaging apparatus according to any of claims 8 to 11 wherein the photoconductive element further comprises a second charge transport material.

**13.** An electrophotographic imaging apparatus according to claim 12 wherein second charge transport material comprises an electron transport compound.

**14.** An electrophotographic imaging apparatus according to any of claims 8 to 13 further comprising a liquid toner dispenser.

**15.** An electrophotographic imaging process comprising;

(a) applying an electrical charge to a surface of an organophotoreceptor comprising an electrically conductive

substrate and a photoconductive element on the electrically conductive substrate, the photoconductive element comprising

(i) a charge transport material having the formula

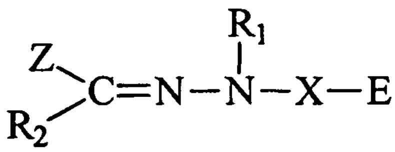

where $R_1$ and $R_2$ are, independently, H, an alkyl group, an alkaryl group, or an aryl group;
X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;
E is an epoxy group; and
Z comprises a phenothiazine group, a phenoxazine group, a phenoxathiin group, a dibenzo(1,4)dioxin group, a thianthrene group, or a phenazine group; and
(ii) a charge generating compound.

(b) imagewise exposing the surface of the organophotoreceptor to radiation to dissipate charge in selected areas and thereby form a pattern of charged and uncharged areas on the surface;
(c) contacting the surface with a toner to create a toned image; and
(d) transferring the toned image to substrate.

**16.** An electrophotographic imaging process according to claim 15 wherein X is a $CH_2$ group.

**17.** An electrophotographic imaging process according to either of claims 15 and 16 wherein Z is a phenothiazine group.

**18.** An electrophotographic imaging process according to any of claims 15 to 17 wherein the charge transport material has a formula selected from the group consisting of the following:

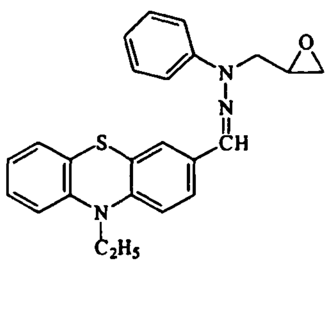

,

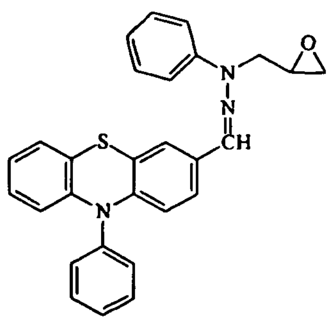

.

**19.** An electrophotographic imaging process according to any of claims 15 to 18 wherein the photoconductive element further comprises a second charge transport material.

**20.** An electrophotographic imaging process according to claim 19 wherein the second charge transport material comprises an electron transport compound.

**21.** An electrophotographic imaging process according to any of claims 15 to 20 wherein the photoconductive element further comprises a binder.

**22.** An electrophotographic imaging process according to any of claims 15 to 21 wherein the toner comprises a liquid toner comprising a dispersion of colorant particles in an organic liquid.

**23.** A charge transport material having the formula

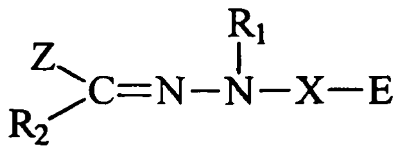

where $R_1$ and $R_2$ are, independently, H, an alkyl group, an alkaryl group, or an aryl group;
X is a linking group having the formula $-(CH_2)_m-$, branched or linear, where m is an integer between 1 and 20, inclusive, and one or more of the methylene groups is optionally replaced by O, S, C=O, O=S=O, a heterocyclic group, an aromatic group, urethane, urea, an ester group, a $NR_3$ group, a $CHR_4$ group, or a $CR_5R_6$ group where $R_3$, $R_4$, $R_5$, and $R_6$ are, independently, H, hydroxyl group, thiol group, an alkyl group, an alkaryl group, a heterocyclic group, or an aryl group;
E is an epoxy group; and
Z comprises a phenothiazine group, a phenoxazine group, a phenoxathiin group, a dibenzo(1,4)dioxin group, a thianthrene group, or a phenazine group.

**24.** A charge transport material according to claim 23 wherein X is a $CH_2$ group.

**25.** A charge transport material according to either of claims 23 and 24 wherein Z is a phenothiazine group.

**26.** A charge transport material according to any of claims 23 to 25 wherein the charge transport material has a formula selected from the group consisting of the following:

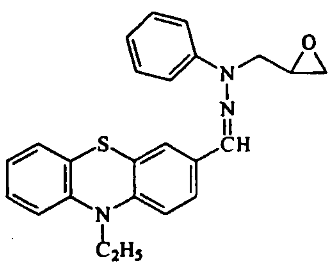
,
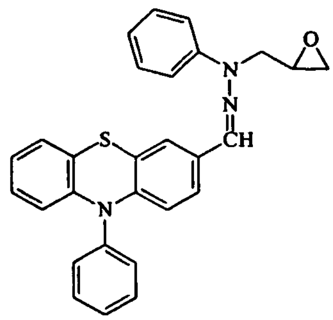
.

**Patentansprüche**

**1.** Organophotorezeptor umfassend ein elektrisch leitfähiges Substrat und ein photoleitfähiges Element auf dem elektrisch leitfähigen Substrat, wobei das photoleitfähige Element:

(a) ein Ladungstransportmaterial mit der Formel

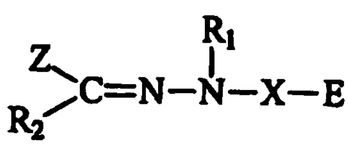

worin $R_1$ und $R_2$ unabhängig H, eine Alkylgruppe, eine Alkarylgruppe oder eine Arylgruppe sind;
X eine verzweigte oder gerade Verbindungsgruppe mit der Formel $-(CH_2)_m$,- ist, worin m eine ganze Zahl zwischen 1 und 20 einschließlich ist und eine oder mehr Methylengruppen gegebenenfalls durch O, S, C=O, O=S=O, eine heterocyclische Gruppe, eine aromatische Gruppe, Urethan, Harnstoff, eine Estergruppe, eine Gruppe $NR_3$, eine Gruppe $CHR_4$ oder eine Gruppe $CR_5R_6$ ersetzt sind, worin $R_3$, $R_4$, $R_5$ und $R_6$ unabhängig H, eine Hydroxygruppe, Thiolgruppe, eine Alkylgruppe, eine Alkarylgruppe, eine heterocyclische Gruppe oder eine Arylgruppe sind;
E eine Epoxygruppe ist und
Z eine Phenothiazingruppe, eine Phenoxazingruppe, eine Phenoxathiingruppe, eine Dibenzo(1,4)dioxingruppe, eine Thianthrengruppe oder eine Phenazingruppe umfaßt, und
(b) eine ladungserzeugende Verbindung umfaßt.

**2.** Organophotorezeptor gemäß Anspruch 1, wobei X eine $CH_2$-Gruppe ist.

**3.** Organophotorezeptor gemäß einem der Ansprüche 1 und 2, wobei Z eine Phenothiazingruppe ist.

**4.** Organophotorezeptor gemäß einem vorangehenden Anspruch, wobei das Ladungstransportmaterial eine Formel aufweist, die aus der aus dem Folgenden bestehenden Gruppe ausgewählt ist:

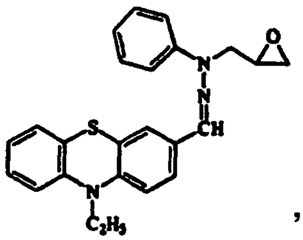
,
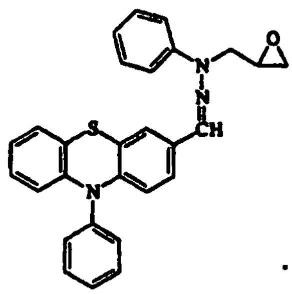
.

**5.** Organophotorezeptor gemäß einem vorangehenden Anspruch, wobei das photoleitfähige Element weiter ein zweites Ladungstransportmaterial umfaßt.

**6.** Organophotorezeptor gemäß Anspruch 5, wobei das zweite Ladungstransportmaterial eine Elektronentransportverbindung umfaßt.

**7.** Organophotorezeptor gemäß einem vorangehenden Anspruch, wobei das photoleitfähige Element weiter ein Bindemittel umfaßt.

**8.** Elektrophotographisches Bilderzeugungsgerät umfassend:

(a) eine mit Licht bilderzeugende Komponente und (b) einen zum Empfangen von Licht aus der mit Licht bilderzeugenden Komponente ausgerichteten Organophotorezeptor, wobei der Organophotorezeptor ein elektrisch leitfähiges Substrat und ein photoleitfähiges Element auf dem elektrisch leitfähigen Substrat umfaßt und das photoleitfähige Element:

(i) ein Ladungstransportmaterial mit der Formel

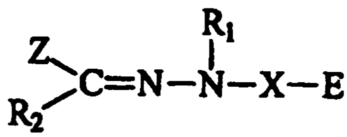

worin $R_1$ und $R_2$ unabhängig H, eine Alkylgruppe, eine Alkarylgruppe oder eine Arylgruppe sind;
X eine verzweigte oder gerade Verbindungsgruppe mit der Formel $-(CH_2)_m$- ist, worin m eine ganze Zahl zwischen 1 und 20 einschließlich ist und eine oder mehr Methylengruppen gegebenenfalls durch O, S,

C=O, O=S=O, eine heterocyclische Gruppe, eine aromatische Gruppe, Urethan, Harnstoff, eine Estergruppe, eine Gruppe $NR_3$, eine Gruppe $CHR_4$ oder eine Gruppe $CR_5R_6$ ersetzt sind, worin $R_3$, $R_4$, $R_5$ und $R_6$ unabhängig H, eine Hydroxygruppe, Thiolgruppe, eine Alkylgruppe, eine Alkarylgruppe, eine heterocyclische Gruppe oder eine Arylgruppe sind;
E eine Epoxygruppe ist und
Z eine Phenothiazingruppe, eine Phenoxazingruppe, eine Phenoxathüngruppe, eine Dibenzo(1,4)dioxingruppe, eine Thianthrengruppe oder eine Phenazingruppe umfaßt, und

(b) eine ladungserzeugende Verbindung umfaßt.

**9.** Elektrophotographisches Bilderzeugungsgerät gemäß Anspruch 8, wobei X eine $CH_2$-Gruppe ist.

**10.** Elektrophotographisches Bilderzeugungsgerät gemäß einem der Ansprüche 8 und 9, wobei Z eine Phenothiazingruppe ist.

**11.** Elektrophotographisches Bilderzeugungsgerät gemäß einem der Ansprüche 8 bis 10, wobei das Ladungstransportmaterial eine Formel aufweist, die aus der aus dem Folgenden bestehenden Gruppe ausgewählt ist:

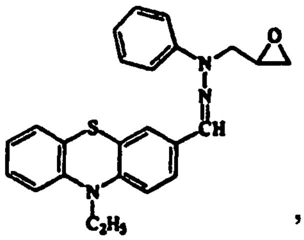
,
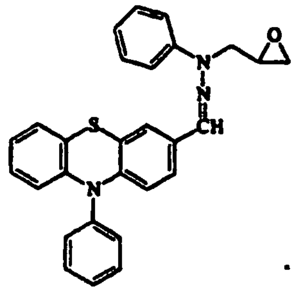
.

**12.** Elektrophotographisches Bilderzeugungsgerät gemäß einem der Ansprüche 8 bis 11, wobei das photoleitfähige Element weiter ein zweites Ladungstransportmaterial umfaßt.

**13.** Elektrophotographisches Bilderzeugungsgerät gemäß Anspruch 12, wobei das zweite Ladungstransportmaterial eine Elektronentransportverbindung umfaßt.

**14.** Elektrophotographisches Bilderzeugungsgerät gemäß einem der Ansprüche 8 bis 13, das weiter eine Flüssigtonerabgabevorrichtung umfaßt.

**15.** Elektrophotographisches Bilderzeugungsverfahren umfassend:

(a) das Anlegen einer elektrischen Ladung an eine Oberfläche eines Organophotorezeptors, der ein elektrisch leitfähiges Substrat und ein photoleitfähiges Element auf dem elektrisch leitfähigen Substrat umfaßt, wobei das photoleitfähige Element

(i) ein Ladungstransportmaterial mit der Formel

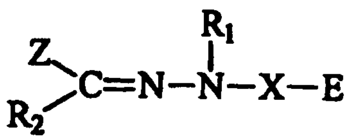

worin $R_1$ und $R_2$ unabhängig H, eine Alkylgruppe, eine Alkarylgruppe oder eine Arylgruppe sind;
X eine verzweigte oder gerade Verbindungsgruppe mit der Formel $-(CH_2)_m-$ ist, worin m eine ganze Zahl zwischen 1 und 20 einschließlich ist und eine oder mehr Methylengruppen gegebenenfalls durch O, S, C=O, O=S=O, eine heterocyclische Gruppe, eine aromatische Gruppe, Urethan, Harnstoff, eine Estergrup-

pe, eine Gruppe $NR_3$, eine Gruppe $CHR_4$ oder eine Gruppe $CR_5R_6$ ersetzt sind, worin $R_3$, $R_4$, $R_5$ und $R_6$ unabhängig H, eine Hydroxygruppe, Thiolgruppe, eine Alkylgruppe, eine Alkarylgruppe, eine heterocyclische Gruppe oder eine Arylgruppe sind;
E eine Epoxygruppe ist und
Z eine Phenothiazingruppe, eine Phenoxazingruppe, eine Phenoxathüngruppe, eine Dibenzo(1,4)dioxingruppe, eine Thianthrengruppe oder eine Phenazingruppe umfaßt, und
(ii) eine ladungserzeugende Verbindung umfaßt,

(b) das bildweise Aussetzen der Oberfläche des Organophotorezeptors einer Strahlung unter Ableiten von Ladung in ausgewählten Bereichen und **dadurch** Bilden eines Musters aus geladenen und ungeladenen Bereichen auf der Oberfläche;
(c) das In-Kontakt-Bringen der Oberfläche mit einem Toner unter Erzeugen eines Tonerbildes, und
(d) das Übertragen des Tonerbildes auf ein Substrat.

**16.** Elektrophotographisches Bilderzeugungsverfahren gemäß Anspruch 15, wobei X eine $CH_2$-Gruppe ist.

**17.** Elektrophotographisches Bilderzeugungsverfahren gemäß einem der Ansprüche 15 und 16, wobei Z eine Phenothiazingruppe ist.

**18.** Elektrophotographisches Bilderzeugungsverfahren gemäß einem der Ansprüche 15 bis 17, wobei das Ladungstransportmaterial eine Formel aufweist, die aus der aus dem Folgenden bestehenden Gruppe ausgewählt ist:

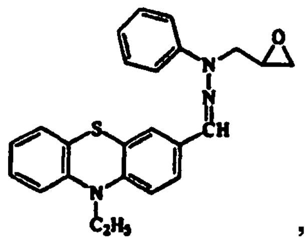
,
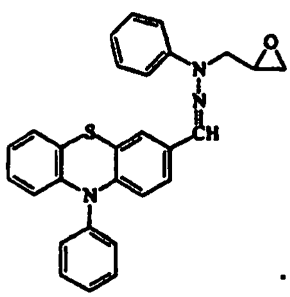
.

**19.** Elektrophotographisches Bilderzeugungsverfahren gemäß einem der Ansprüche 15 bis 18, wobei das photoleitfähige Element weiter ein zweites Ladungstransportmaterial umfaßt.

**20.** Elektrophotographisches Bilderzeugungsverfahren gemäß Anspruch 19, wobei das zweite Ladungstransportmaterial eine Elektronentransportverbindung umfaßt.

**21.** Elektrophotographisches Bilderzeugungsverfahren gemäß einem der Ansprüche 15 bis 20, wobei das photoleitfähige Element weiter ein Bindemittel umfaßt.

**22.** Elektrophotographisches Bilderzeugungsverfahren gemäß einem der Ansprüche 15 bis 21, wobei der Toner einen Flüssigtoner umfaßt, der eine Dispersion von Farbmittelteilchen in einer organischen Flüssigkeit umfaßt.

**23.** Ladungstransportmaterial mit der Formel

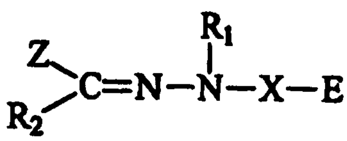

worin $R_1$ und $R_2$ unabhängig H, eine Alkylgruppe, eine Alkarylgruppe oder eine Arylgruppe sind;
X eine verzweigte oder gerade Verbindungsgruppe mit der Formel $-(CH_2)_m-$ ist, worin m eine ganze Zahl zwischen 1 und 20 einschließlich ist und eine oder mehr Methylengruppen gegebenenfalls durch O, S, C=O, O=S=O, eine

heterocyclische Gruppe, eine aromatische Gruppe, Urethan, Harnstoff, eine Estergruppe, eine Gruppe $NR_3$, eine Gruppe $CHR_4$ oder eine Gruppe $CR_5R_6$ ersetzt sind, worin $R_3$, $R_4$, $R_5$ und $R_6$ jeweils H, eine Hydroxygruppe, Thiolgruppe, eine Alkylgruppe, eine Alkarylgruppe, eine heterocyclische Gruppe oder eine Arylgruppe sind;
E eine Epoxygruppe ist und
Z eine Phenothiazingruppe, eine Phenoxazingruppe, eine Phenoxathiingruppe, eine Dibenzo(1,4)dioxingruppe, eine Thianthrengruppe oder eine Phenazingruppe umfaßt.

**24.** Ladungstransportmaterial gemäß Anspruch 23, wobei X eine $CH_2$-Gruppe ist.

**25.** Ladungstransportmaterial gemäß einem der Ansprüche 23 und 24, wobei Z eine Phenothiazingruppe ist.

**26.** Ladungstransportmaterial gemäß einem der Ansprüche 23 bis 25, wobei das Ladungstransportmaterial eine Formel aufweist, die aus der aus dem Folgenden bestehenden Gruppe ausgewählt ist:

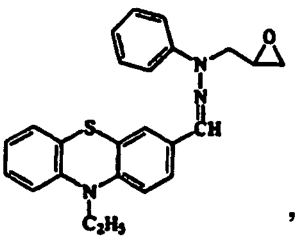
,
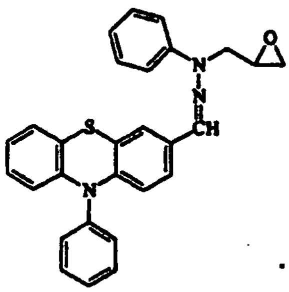
.

## Revendications

**1.** Organophotorécepteur comprenant un substrat électriquement conducteur et un élément photoconducteur sur le substrat électriquement conducteur, l'élément photoconducteur comprenant :

(a) un matériau de transport de charge ayant la formule

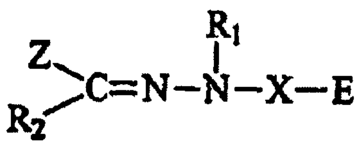

dans laquelle $R_1$ et $R_2$ sont, indépendamment, H, un groupe alkyle, un groupe aralkyle, ou un groupe aryle ;
X est un groupe de liaison ayant la formule $-(CH_2)_m-$, linéaire ou ramifié, dans laquelle m est un nombre entier entre 1 et 20, inclus, et un ou plus des groupes méthylènes est facultativement remplacé par O, S, C=O, O=S=O, un groupe hétérocyclique, un groupe aromatique, un groupe uréthane, un groupe urée un groupe ester, un groupe $NR_3$, un groupe $CHR_4$, ou un groupe $CR_5R_6$ où $R_3$, $R_4$, $R_5$, et $R_6$ sont, indépendamment, H, un groupe hydroxyle, un groupe thiol, un groupe alkyle, un groupe alkaryle, un groupe hétérocyclique, ou un groupe aryle ;
E est un groupe époxy ; et
Z comprend un groupe phénothiazine, un groupe phénoxazine, un groupe phénoxathiine, un groupe dibenzo (1,4)dioxine, un groupe thianthrène, ou un groupe phénazine ; et
(b) un composé de génération de charge.

**2.** Organophotorécepteur selon la revendication 1, dans lequel X est un groupe $CH_2$.

**3.** Organophotorécepteur selon l'une ou l'autre des revendications 1 et 2, dans lequel Z est un groupe phénothiazine.

**4.** Organophotorécepteur selon l'une quelconque des revendications précédentes, dans lequel le matériau de transport

de charge a une formule choisie dans le groupe constitué par les formules suivantes :

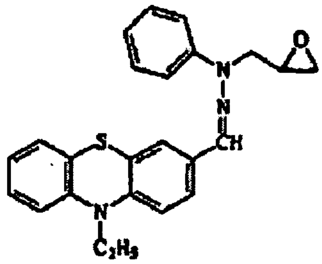

,

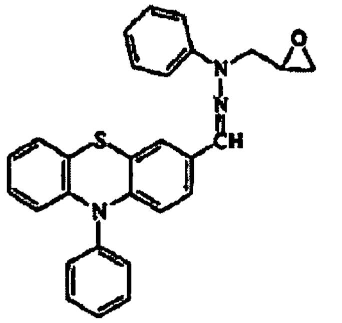

.

**5.** Organophotorécepteur selon l'une quelconque des revendications précédentes, dans lequel l'élément photoconducteur comprend en outre un second matériau de transport de charges.

**6.** Organophotorécepteur selon la revendication 5, dans lequel le second matériau de transport de charges comprend un composé de transport d'électrons.

**7.** Organophotorécepteur selon l'une quelconque des revendications précédentes, dans lequel l'élément photoconducteur comprend en outre un liant.

**8.** Appareil d'imagerie électrophotographique comprenant :

(a) un composant de formation d'image lumineuse ; et
(b) un organophotorécepteur orienté pour recevoir la lumière du composant de formation d'image lumineuse, l'organophotorécepteur comprenant un substrat électriquement conducteur et un élément photoconducteur sur le substrat électriquement conducteur, l'élément photoconducteur comprenant :

(i) un matériau de transport de charge ayant la formule

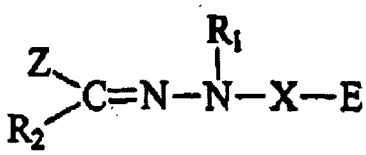

dans laquelle $R_1$ et $R_2$ sont, indépendamment, H, un groupe alkyle, un groupe aralkyle, ou un groupe aryle ;
X est un groupe de liaison ayant la formule $-(CH_2)_m-$, linéaire ou ramifié, dans laquelle m est un nombre entier entre 1 et 20, inclus, et un ou plus des groupes méthylènes est facultativement remplacé par O, S, C=O, O=S=O, un groupe hétérocyclique, un groupe aromatique, un groupe uréthane, un groupe urée, un groupe ester, un groupe $NR_3$, un groupe $CHR_4$, ou un groupe $CR_5R_6$ où $R_3$, $R_4$, $R_5$, et $R_6$ sont, indépendamment, H, un groupe hydroxyle, un groupe thiol, un groupe alkyle, un groupe alkaryle, un groupe hétérocyclique, ou un groupe aryle ;
E est un groupe époxy ; et
Z comprend un groupe phénothiazine, un groupe phénoxazine, un groupe phénoxathiine, un groupe dibenzo (1,4)dioxine, un groupe thianthrène, ou un groupe phénazine ; et
(ii) un composé de génération de charge.

**9.** Appareil d'imagerie électrophotographique selon la revendication 8, dans lequel X est un groupe $CH_2$.

**10.** Appareil d'imagerie électrophotographique selon l'une ou l'autre des revendications 8 et 9, dans lequel Z est un groupe phénothiazine.

**11.** Appareil d'imagerie électrophotographique selon l'une quelconque des revendications 8 à 10, dans lequel le matériau de transport de charges a une formule choisie dans le groupe constitué par les formules suivantes :

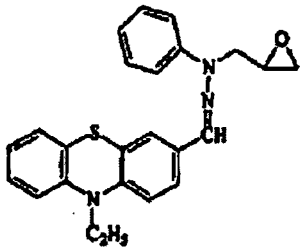

,

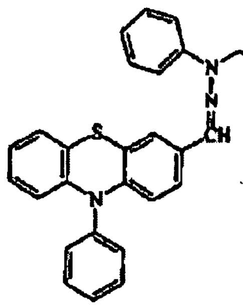

.

**12.** Appareil d'imagerie électrophotographique selon l'une quelconque des revendications 8 à 11, dans lequel l'élément photoconducteur comprend en outre un second matériau de transport de charges.

**13.** Appareil d'imagerie électrophotographique selon la revendication 12, dans lequel le second matériau de transport de charges comprend un composé de transport d'électrons.

**14.** Appareil d'imagerie électrophotographique selon l'une quelconque des revendications 8 à 13, comprenant en outre un distributeur de toner liquide.

**15.** Processus d'imagerie électrophotographique comprenant :

(a) l'application d'une charge électrique à une surface d'un organophotorécepteur comprenant un substrat électriquement conducteur et un élément photoconducteur sur le substrat électriquement conducteur, l'élément photoconducteur comprenant :

(i) un matériau de transport de charge ayant la formule

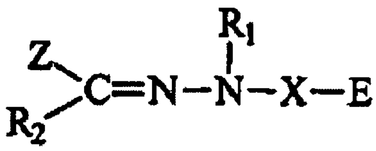

dans laquelle $R_1$ et $R_2$ sont, indépendamment, H, un groupe alkyle, un groupe alkaryle, ou un groupe aryle ;
X est un groupe de liaison ayant la formule $-(CH_2)_m-$, linéaire ou ramifié, dans laquelle m est un nombre entier entre 1 et 20, inclus, et un ou plus des groupes méthylènes est facultativement remplacé par O, S, C=O, O=S=O, un groupe hétérocyclique, un groupe aromatique, un groupe uréthane, un groupe urée un groupe ester, un groupe $NR_3$, un groupe $CHR_4$, ou un groupe $CR_5R_6$ où $R_3$, $R_4$, $R_5$, et $R_6$ sont, indépendamment, H, un groupe hydroxyle, un groupe thiol, un groupe alkyle, un groupe alkaryle, un groupe hétérocyclique, ou un groupe aryle ;
E est un groupe époxy ; et
Z comprend un groupe phénothiazine, un groupe phénoxazine, un groupe phénoxathiine, un groupe dibenzo (1,4)dioxine, un groupe thianthrène, ou un groupe phénazine ; et
(ii) un composé de génération de charge ;

(b) l'exposition au motif d'une image de la surface de l'organophotorécepteur à une radiation pour dissiper la charge dans des zones choisies et ainsi former un motif de zones chargées et non chargées sur la surface ;
(c) la mise en contact de la surface avec un toner pour créer une image teintée ; et
(d) le transfert de l'image teintée au substrat.

16. Processus d'imagerie électrophotographique selon la revendication 15, dans lequel X est un groupe $CH_2$.

17. Processus d'imagerie électrophotographique selon l'une ou l'autre des revendications 15 et 16, dans lequel Z est un groupe phénothiazine.

18. Processus d'imagerie électrophotographique selon l'une quelconque des revendications 15 à 17, dans lequel le matériau de transport de charge a une formule choisie dans le groupe constitué par les formules suivantes :

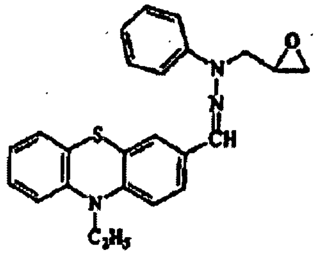

,

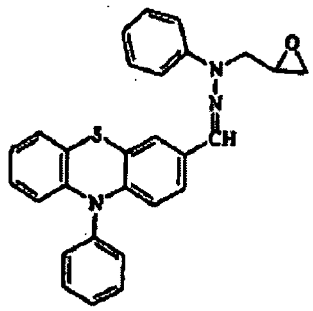

.

19. Processus d'imagerie électrophotographique selon l'une quelconque des revendications 15 à 18, dans lequel l'élément photoconducteur comprend en outre un second matériau de transport de charges.

20. Processus d'imagerie électrophotographique selon la revendication 19, dans lequel le second matériau de transport de charge comprend un composé de transport d'électrons.

21. Processus d'imagerie électrophotographique selon l'une quelconque des revendications 15 à 20, dans lequel l'élément photoconducteur comprend en outre un liant.

22. Processus d'imagerie électrophotographique selon l'une quelconque des revendications 15 à 21, dans lequel le toner comprend un toner liquide comprenant une dispersion de particules de colorant dans un liquide organique.

23. Matériau de transport de charge ayant la formule

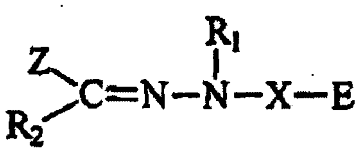

dans laquelle $R_1$ et $R_2$ sont, indépendamment, H, un groupe alkyle, un groupe alkaryle, ou un groupe aryle ;
X est un groupe de liaison ayant la formule $-(CH_2)_m-$, linéaire ou ramifié, dans laquelle m est un nombre entier entre 1 et 20, inclus, et un ou plus des groupes méthylènes est facultativement remplacé par O, S, C=O, O=S=O, un groupe hétérocyclique, un groupe aromatique, un groupe uréthane, un groupe urée un groupe ester, un groupe $NR_3$, un groupe $CHR_4$, ou un groupe $CR_5R_6$ où $R_3$, $R_4$, $R_5$, et $R_6$ sont, indépendamment, H, un groupe hydroxyle, un groupe thiol, un groupe alkyle, un groupe aralkyle, un groupe hétérocyclique, ou un groupe aryle ;
E est un groupe époxy ; et
Z comprend un groupe phénothiazine, un groupe phénoxazine, un groupe phénoxathiine, un groupe dibenzo(1,4) dioxine, un groupe thianthrène, ou un groupe phénazine.

24. Matériau de transport de charges selon la revendication 23, dans lequel X est un groupe $CH_2$.

25. Matériau de transport de charges selon l'une ou l'autre des revendications 23 et 24, dans lequel Z est un groupe phénothiazine.

**26.** Matériau de transport de charges selon l'une quelconque des revendications 23 à 25, dans lequel le matériau de transport de charges a une formule choisie dans le groupe constitué par les formules suivantes :

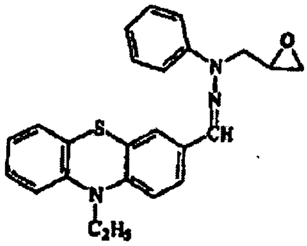

,

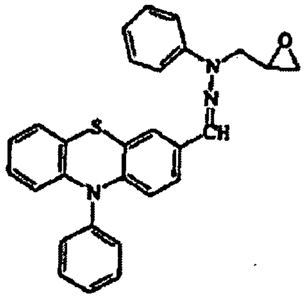

.